## Europäisches Patentamt

## European Patent Office

## Office européen des brevets

(11) Veröffentlichungsnummer: **0 134 251**
**B1**

(12) # EUROPÄISCHE PATENTSCHRIFT

(45) Veröffentlichungstag der Patentschrift:
14.10.87

(51) Int. Cl.⁴: **A 61 B 17/32**, A 61 B 17/28

(21) Anmeldenummer: **83106767.3**

(22) Anmeldetag: **09.07.83**

(54) **Medizinische Zange.**

(30) Priorität: **16.04.83 DE 8311392 U**

(43) Veröffentlichungstag der Anmeldung:
**20.03.85 Patentblatt 85/12**

(45) Bekanntmachung des Hinweises auf die Patenterteilung:
**14.10.87 Patentblatt 87/42**

(84) Benannte Vertragsstaaten:
**AT BE CH DE FR GB IT LI NL SE**

(56) Entgegenhaltungen:
**DE-B-2 808 911**
**GB-A-702 683**
**US-A-2 146 954**
**US-A-3 835 860**

(73) Patentinhaber: **Storz- Endoskop GmbH,
Kreuzgutweg 22, CH- 8207 Schaffhausen (CH)**

(72) Erfinder: **Storz, Karl, Auf dem Schildrain 39, D-7200
Tuttlingen (DE)**

(74) Vertreter: **Wenzel, Joachim, Dipl.- Ing.,
Hauptmannsreute 46, D-7000 Stuttgart 1 (DE)**

LIBER, STOCKHOLM 1987

## Beschreibung

Die Erfindung betrifft eine medizinische Stanzzange nach dem Oberbegriff des Anspruchs 1.

Eine bekannte Stanzzange dieser Art ist zum Abtragen von Knochen und Knorpel bei Operationen im Kopf- und Wirbelsäulenbereich bestimmt. Hierbei ist eine Stange mit einer Stanzbacke axial unbeweglich in einer Hülse befestigt, während ein die Hülse ummantelndes Rohr axial bewegt wird, um die Stanzbacken gegeneinander zu führen (DE-AS 28 08 911).

Der Erfindung liegt die Aufgabe zugrunde, diese bekannte medizinische Stanzzange so auszubilden, daß sie zum Abtragen von Gewebe, insbesondere im Kniegelenk bei endoskopischen Operationen geeignet ist, wobei das Zahnstangengetriebe zur Betätigung des Stanzwerkzeuges über eine Welle angetrieben werden soll, wie dies bei einer anderen Vorrichtung dieser allgemeinen Art bekannt ist, bei der aber die Betätigung durch die beiden Griffschenkel der Zange mit Fingerringen fehlt (vgl. GB-A-702 863). Hierzu soll eine noch größere Kraftübersetzung erzielt werden können als dies bekannt ist.

Zur Lösung dieser Aufgabe sind die Merkmale des kennzeichnenden Teils des Anspruchs 1 vorgesehen.

Durch die Erfindung wird somit ein Getriebe zwischen die beiden Griffschenkel eingebaut, das eine gewisse Ähnlichkeit mit einem Kniehebel-Gelenkgetriebe aufweist. Auf diese Weise ist die Kraftübersetzung noch größer als in dem bekannten Falle.

In weiterer Ausgestaltung der Erfindung sind die kennzeichnenden Merkmale des Anspruchs 2 vorgesehen. Je größer der Abstandsunterschied der beiden Gelenkverbindungen ist, desto größer ist die Kraftverstärkung.

Weitere Vorteile und Einzelheiten der Erfindung ergeben sich aus der nun folgenden Beschreibung eines Ausführungsbeispiels unter Hinweis auf die Zeichnung. In dieser zeigen:

Fig. 1 eine Seitenansicht auf die Ausführungsform mit geschlossenem Stanzwerkzeug;

Fig. 2 das Gleiche wie Fig. 1 jedoch mit geöffnetem Stanzwerkzeug;

Fig. 3 eine Draufsicht nur auf das Werkzeug für sich allein nach Fig. 2;

Fig. 4 eine Teil-Seitenansicht, teilweise geschnitten, und

Fig. 5 eine Ansicht in Richtung X gemäß Fig. 2.

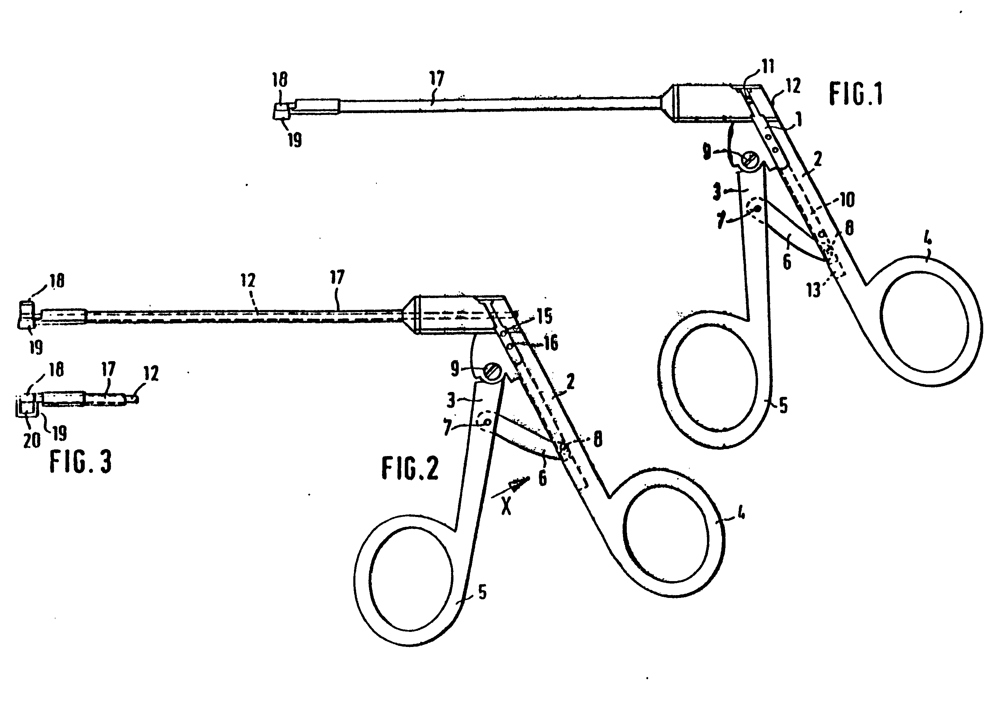

Fig. 1 zeigt eine Zange mit den beiden Griffschenkeln 2 und 3, die durch die üblichen Fingerringe 4, 5 in bekannter Weise betätigt werden. An dem feststehenden Griffschenkel 2, sieht man links eine Gelenkschraube 9, mittels der der Griffschenkel 3 in bekannter Weise bewegbar ist. In dem bewegbaren Griffschenkel 3 sieht man unterhalb der Gelenkschraube 9 im Punkt 7 den Hebel 6 gelenkig befestigt. Am anderen Ende des Hebels 6 ist ebenfalls ein Gelenk 8 vorhanden, das jedoch nicht starr in dem Griffschenkel 3 angeordnet ist, sondern an der Verlängerung 10 gelenkig befestigt ist, die in einer Ausnehmung 13 angeordnet ist, in der sie in Längsrichtung des Griffschenkels 2 hin- und herbewegbar ist. Da diese Verlängerung 10 innerhalb des Schenkels 2 in der erwähnten Ausnehmung 13 liegt, ist sie nicht sichtbar, sondern wird durch unterbrochene Linien angedeutet.

Am oberen Ende ist diese Verlängerung 10 mit der Zahnstange 1 ebenfalls in Längsrichtung des Schenkels 2 fest verbunden, die jedoch außen an dem Schenkel 2 anliegt und deshalb sichtbar ist. Die Zähne dieser Zahnstange 1 sind hier nicht sichtbar, weil sie nach innen gerichtet sind. Oben sieht man am Ende der Zahnstange das Zahnrad 11 ein wenig durch eine Lücke in dem Griffschenkel. Rechts davon sieht man das Ende der Welle 12, auf der das Zahnrad 11 sitzt. Diese Welle setzt sich nach links innerhalb des Zangenschaftes 17 bis zu dem Stanzwerkzeug 18, 19 am distalen Ende zur Betätigung desselben fort. Dabei ist die Welle 12 mit dem bewegbaren Teil 18 des Stanzwerkzeuges fest verbunden und bewegt diesen so, wie dies später noch im einzelnen erläutert wird.

Fig. 2 zeigt die geöffnete Stellung, bei der die beiden Fingerringe 4 und 5 also auseinander gedrückt sind. Gegenüber der Fig. 1 ergibt sich hierbei folgendes:

Der Hebel 6 bewegt sich in den beiden Gelenken 7 und 8. Da nun das Gelenk 7 mit dem Griffschenkel 3 fest verbunden ist, bewirkt das Gelenk 8 über die Verlängerung 10 eine Verschiebung der Zahnstange 1 nach links oben in Längsrichtung des Griffschenkels 2 innerhalb der Ausnehmung 13.

In dieser Fig. 2 sieht man auch daß die Zahnstange 1 mittels zweier Nieten 15, 16 mit der innenliegenden Verlängerung 10 fest verbunden ist, wobei sich die Nieten in einem hier nicht sichtbaren Schlitz für diese beiden Nieten bewegt. Dadurch ist die erwähnte obere Lücke durch die Zahnstange 1 geschlossen worden und das bewegliche Teil 18 auf der Welle 12 ist etwa im rechten Winkel nach oben geschwenkt. Dadurch ist das Zahnrad 11 in dieser Ansicht überhaupt nicht zu sehen. Es steht aber mit den Zähnen der Zahnstange 1 an der dieser Ansicht gegenüberliegenden Seite im Eingriff.

Fig. 3 zeigt die Draufsicht auf das Stanzwerkzeug am distalen Ende. Daraus kann man erkennen, daß das bewegbare Teil 18 etwa im rechten Winkel zu dem feststehenden Teil 19 des Stanzwerkzeugesliegt, das in der Mitte eine Ausnehmung 20 aufweist.

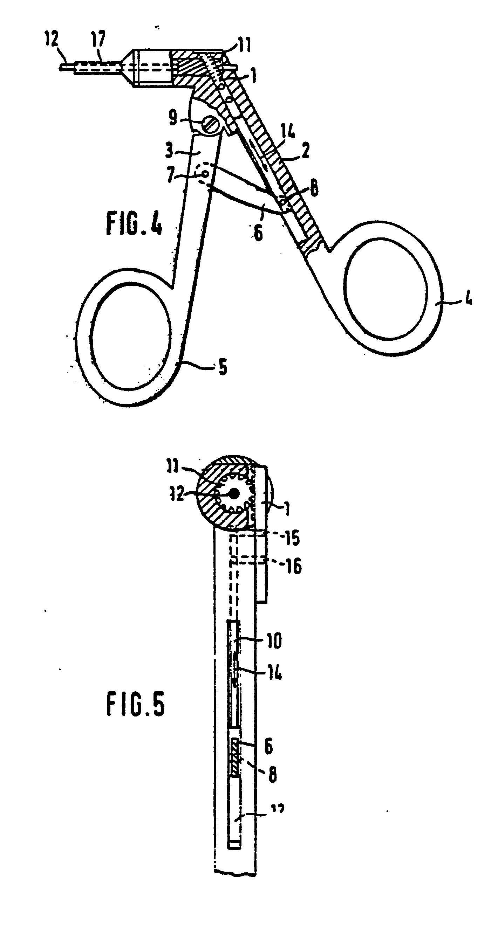

Fig. 4 zeigt die beiden Schenkel 2, 3 teilweise geschnitten, sodaß die Funktion und Wirkungsweise hier vollständig erläutert werden kann. Die Ausnehmung 13 ist in Richtung des Pfeiles X nach Fig. 2 eingebracht und hier offengelegt. Dadurch sieht man das Gelank 8, das mit der Verlängerung 10 in Gelenkverbindung steht, welches Teil in Richtung des Pfeiles 14 in beiden Richtungen in dieser Ausnehmung geführt ist. Am Ende der Verlängerung 10 sieht man nunmehr deutlich die Zahnstange 1 mit schrägliegenden Zähnen, die in das Zahnrad 11 im Eingriff stehen, wobei es sich um eine Schrägverzahnung handelt. Dies ist zweckmäßig bzw. erforderlich, damit zwischen der Welle 15 und der Richtung der Pfeile 14 ein gewisser Winkel vorhanden sein kann, der vom rechten Winkel abweicht. Bei Gradverzahnung müßte der rechte Winkel vorhanden sein, was aber der üblichen Bauform dieser medizinischen Zangen im allgemeinen nicht entspricht.

Fig. 4 zeigt die Offenstellung der Zange nach Fig. 2. Wenn aus dieser Stellung heraus die beiden Fingerringe 4, 5 zusammengedrückt werden, wird das Gelenk 8 die Verlängerung 10 und somit die Zahnstange 1 mit großer Kraft in Richtung der Pfeile 14 nach unten drücken. Je größer nun die Abstandsdifferenz zwischen dem Gelenk 7 und 9 sowie dem Gelenk 8 und 9 ist, desto größer wird die Kraft nach Art eines Knie-Hebel-Gelenkes sein.

Die Erfindung ist aber nicht auf diese Ausführungsform beschränkt, sondern es besteht zum Beispiel auch die Möglichkeit, daß das feste Gelenk 7 weiter unten in der Nähe des Fingerringes 5 angeordnet ist und das Gelenk 8 weiter oben in der Nähe der Zahnstange 1. Dann wäre allerdings die Wirkrichtung umgekehrt. Dies wäre aber dadurch zu kompensieren, daß man die Zahnstange 1 auf der gegenüberliegenden Seite anbringen würde. Auf diese Weise würde die Verlängerung 10 wesentlich kürzer ausfallen.

In Fig. 5 ist die Ansicht in Richtung X auf den Griffschenkel 2 nach Fig. 2 dargestellt. Man erkennt hier deutlich, daß die Verlängerung 10 in der Ausnehmung 13 des Schenkels 2 angeordnet ist. Sie ist auch in der Ausnehmung 13 in Richtung des Pfeiles 14 geführt.

Weiter oben sieht man, daß die Zahnstange 1 rechts außen an den Griffschenkel 2 angebracht ist. Wie früher schon erwähnt, ist die Verbindung zwischen der Zahnstange 1 und der Verlängerung 10 durch die beiden Nieten 15 und 16 nach Fig. 2 hergestellt, was hier der Klarheit wegen nicht dargestellt ist. Der Fachmann kann sich aber leicht vorstellen, daß für die beiden Nieten 15 und 16 eine entsprechende Nut in dem Griffschenkel 2 vorhanden ist, in der sich diese Nieten in Richtung des Pfeiles 14 bewegen können. Am oberen Ende der Zahnstange 1 sind die Zähne nach links gerichtet im Eingriff mit dem Zahnrad 11 nunmehr sichtbar. Das Zahnrad 11 sitzt auf der Welle 12, welche hier geschnitten dargestellt ist.

Wie anhand der Fig. 4 schon erläutert wurde, besteht die weitere Möglichkeit, das Gelenk 7 nach Figur weiter unten und das Glenk 8 weiter oben in der Nähe der Zahnstange 1 anzuordnen. In diesem Falle ist dann erforderlich, die Zahnstange 1 auf der linken Seite anders als in Fig. 5 anzuordnen. Im übrigen kann die Konstruktion aber die Gleiche bleiben.

Die Darstellung nach Fig. 5 ist gegenüber den vorangegangenen Figuren der Klarheit wegen in vergrößertem Maßstab gezeichnet.

Der Hauptvorteil der Erfindung besteht darin, daß der Arzt wesentlich bequemer als bisher arbeiten kann. Das ist auch dadurch bedingt, daß der Arzt an die Arbeit mit derartigen Zangen gewöhnt ist, die mit den Fingerringen 4, 5 versehen sind.

Ein weiterer Vorteil besteht darin, daß, wie oben schon erwähnt, die Kraftverstärkung durch das Kniehebel-Stangengetriebe praktisch beliebig groß je nach den erwähnten Abmessungen gewählt werden kann.

**Patentansprüche**

1. Medizinische Stanzzange mit einem verschiebbar gelagerten Zahnstangengetriebe zur Betätigung des beweglichen Teils (18) eines Stanzwerkzeuges (18, 19), und zwei mit Fingerringen (4, 5) versehene Griffschenkel (2, 3), dadurch gekennzeichnet, daß bei Verschiebung der Zahnstange (1) dem beweglichen Teil des Stanzwerkzeuges über eine Welle (12) eine Drehbewegung mittels eines Zahnrades (11) übertragen wird, daß die Zahnstange mit dem einen Ende eines Hebels (6) gelenkig verbunden ist, dessen anderes Ende (7) mit einem bewegbaren Griffschenkel (3) gelenkig in Verbindung steht, und daß die Zahnstange an dem anderen Griffschenkel (2) derart verschiebbar gelagert ist, daß die Verschiebung der Zahnstange durch Betätigung des bewegbaren Griffschenkels (3) erfolgt.

2. Medizinische Stanzzange nach Anspruch 1, dadurch gekennzeichnet, daß die eine Gelenkverbindung (7) des Hebels (6) mit dem bewegbaren Griffschenkel (3) in wesentlich größerer Nähe der gelenkigen Verbindung (9) der Griffschenkel (2, 3) miteinander angeordnet ist als die andere gelenkige Verbindung (8) des Hebels mit der Zahnstange.

3. Medizinische Stanzzange nach Anspruch 2, dadurch gekennzeichnet, daß die Zahnstange (1) mit einer Verlängerung (10) versehen ist, die in einer Ausnehmung (13) des anderen Griffschenkels (2) liegt.

4. Medizinische Stanzzange nach Anspruch 1, dadurch gekennzeichnet, daß das Zahnrad (11) auf der Betätigungswelle (12) schräg verzahnt ist.

5. Medizinische Stanzzange nach Anspruch 3, dadurch gekennzeichnet, daß die Zahnstange (1) an dem anderen Griffschenkel (2) außen angeordnet und durch Nieten (15, 16) mit der in der Ausnehmung (13) liegenden Verlängerung (10) durch einen Schlitz des Griffschenkels verbunden ist.

## Claims

1. Medical punching forceps with a displaceably mounted rack and pinion gear for operating the movable part (18) of a punching tool (18, 19) and two gripping members (2, 3) provided with finger rings (4, 5), characterised in that on displacing the rack (1) a rotary movement is transferred by means of a pinion (11) to the movable part of the punching tool by means of a shaft (12), that the rack is connected in articulated manner with one end of a lever (6), whose other end (7) is connected in articulated manner to a movable gripping member (3) and that the rack is so displaceably mounted on the other gripping member (2), that the displacement of the rack takes place by actuating the movable gripping member (3).

2. Medical punching forceps according to claim 1, characterised in that one articulated connection (7) of lever (6) to the movable gripping member (3) is much nearer to the articulated connection (9) of the gripping members (2, 3) to one another than the other articulated connection (8) of the lever to the rack.

3. Punching forceps according to claim 2, characterised in that the rack (1) is provided with an extension (10), which is located in a recess (13) of the other gripping member (2).

4. Punching forceps according to claim 1, characterised in that pinion (11) is spiral-toothed on actuating shaft (12).

5. Medical punching forceps according to claim 3 characterised in that rack (1) is externally arranged on the other gripping member (2) and is connected by rivets (15, 16) to extension (10) located in recess (13) through a slot in the gripping member.

## Revendications

1. Pince médicinale de découpage avec un mécanisme à crémaillère moulé de façon à pouvoir coulisser pour actionner la partie mobile (18) d'un outil de découpage (18, 19), et avec deux branches de poignée (2, 3) munies d'anneaux pour les doigts (4, 5), pince caractérisée en ce que, lors du coulissement de la crémaillère (1), un mouvement de rotation est transmis, au moyen d'une roue dentée (11) et par l'intermédiaire d'un arbre (12) à la partie mobile de l'outil de découpage, la crémaillère étant reliée par articulation à une des extrémités d'un levier (6), dont l'autre extrémité (7) est en liaison par articulation avec une branche mobile (3) de la poignée, et la crémaillère étant moulée de façon à pouvoir coulisser sur l'autre branche (2) de la poignée, de façon que ce coulissement de la crémaillère soit obtenu par actionnement de la branche mobile (3) de la poignée.

2. Pince médicinale de découpage selon la revencication 1, caractérisée en ce que la liaison par articulation (7) du levier (6) avec la branche mobile (3) de la poignée est disposee a une proximité beaucoup plus grande de la liaison par articulation (9) des branches (2, 3) de la poignée que l'autre liaison par articulation (8) du levier avec la crémaillère.

3. Pince médicinale de découpage, selon la revendication 2, caractérisée en ce que la crémaillère (1) est munie d'un prolongement (10) qui est placé dans un évidement (13) de l'autre branche (2) de la poignée.

4. Pince médicinale de découpage selon la revendication 1, caractérisée en ce que la roue dentée (11) sur l'arbre d'actionnement (12) a une denture oblique.

5. Pince médicinale de découpage selon la revendication 3, caractérisée en ce que la crémaillère (1) sur l'autre branche (2) de la poignée est disposée à l'extérieur et réunie, à travers une fente de cette branche de la poignée, par des rivets (15, 16) dans le prolongement (10) placé dans l'évidement (13).

FIG.1

FIG.2

FIG.3

FIG.4

FIG.5